# EUROPEAN PATENT APPLICATION

(11) **EP 0 949 334 A1**
(43) Date of publication of application: **13.10.1999**
(21) Application number: 98933934.6
(22) Date of filing: 24.07.1998
(51) Int. Cl.: C12N 15/57, C07K 14/47, C07K 16/40, C12N 5/06, C12N 9/64, C12P 21/02

(54) **NOVEL SERINE PROTEASE**

(30) Priority: 24.07.1997 JP 21396997
(71) Applicant: SUNTORY LIMITED, Osaka-shi, Osaka 530-8203 (JP)
(72) Inventor: TSURUOKA, Nobuo, Ibaraki-shi, Osaka 567-0827 (JP); YAMASHIRO, Kyoko, Takatsuki-shi, Osaka 569-0852 (JP); YAMAGUCHI, Nozomi-285-79, Shingoryoguchi-cho, Kyoto-shi, Kyoto 603-8146 (JP)
(74) Representative: Stoner, Gerard Patrick
(86) International application number: JP9803324
(87) International publication number: WO9905290

(57) **Abstract**

A serine protease or peptide fragments thereof having an amino acid sequence identical with that of serine protease as shown in SEQ ID NO: 6 or amino acid sequences derived therefrom by deletion or substitution of a part of the same or addition of one or more amino acids thereto.

## Description

### Technical Field

The present invention relates to a novel serine protease, DNA coding therefor, a process for production of said serine protease, and a process for screening physiologically active substances using said serine protease or DNA coding therefor.

### Background Art

Serine proteases are widely present in animals, plants and microorganisms, and are known to he involved in an extremely large number of biological reactions including food digestion, blood coagulation and fibrinolysis, complement activation, hormone production, ovulation and fertilization, phagocytosis, cell growth, development and differentiation, aging and cancer metastasis, particularly in higher animals (Neurath, H., Science, 224, 350-357, 1984).

In recent years, serine proteases have been confirmed to act as a physiologically important functional molecule in the central nervous system as well. For example, known serine proteases occurring in the brain include tissue plasminogen activator (Sappiro, A-D., Madani, R., Huarte, J., Belin, D., Kiss, J.Z., Wohlwent, A. and Vassalli, J-D., J. Clin. Invest., 92, 679-685, 1993), thrombin (Monard, D., Trends Neurosci., 11, 541-544, 1988), human trypsin IV (Wiegand, U., Corbach, S., Minn, A., Kang, J. and Müller-Hill, B., Gene, 136, 167-175, 1993), neuropsin (Chen, Z-L., Yoshida, S., Kato, K., Momota, Y., Suzuki, J., Tanaka, T., Ito, J., Nishino, H., Aimoto, S., Kiyama, H. and Shiosaka, S., J. Neurosci., 15(7), 5088-5097, 1995), and neurosin (Yamashiro, K., Tsuruoka, N., Kodama, S., Tsujimoto, M., Yamamura, Y., Tanaka, T., Nakazato, H. and Yamaguchi, N., Biochim. Biophys. Acta, 1350, 11-14, 1997).

Not only are these serine proteases in the brain involved in the deployment of the neurite outgrowth of neurons, but they are also assumed to be involved in the synapse-formation process with target neurons (Liu, Y., Fields, R.D., Fitzgerald, S., Festoff, B.W. and Nelson, P.G., J. Neurobiol., 25, 325, 1994).

However, the physiological functions of these serine proteases in the brain are essentially unknown. In addition, although it is predicted that many other serine proteases exist that occur in the brain and are responsible for performing important physiological functions, the majority of these are still not identified.

On the other hand, certain types of serine protease proteins in the coagulation, fibrinolysis and complement system have the kringle domains, EGF-like structures, finger structures, γ-carboxyglutamic acid domains, apple domains and other structures on their N-terminus (Furie, B. and Furie, B.C., Cell, 53, 505-518, 1988). Examples of known serine protease proteins having some kringle domains include urokinase, plasminogen activator and plasminogen.

The kringle domains have the ability to bind with fibrin, heparin and lysine analogue (Scanu, A.M. and Edelstein, C., Biochimica. Biophysica. Acta, 1256, 1-12, 1995), and in the blood fibrinolysis system, plasminogen activator has been known to bind the precipitated fibrin by means of its kringle domains, following activation of nearby bound plasmin. Moreover, the angiogenesis inhibitory factor, angiostatin, has been identified to be the kringle domains in a plasminogen molecule (Cao, Y., Ji, R.W., Davidson, D., Scaller, J., Marti, D., Söhndel, S., McCance, S.G., O'Reilly, M.S., Llinás, M. and Folkman, J., J. Biol. Chem., 271, 29461-29467, 1996), and was shown for the first time to have physiological activity as an independent Kringle domain, that provided the first demonstration of the physiological activity as kringle domains alone.

In addition, the existence of a series of protein groups including cyclophilin-C binding protein, speract receptor, complement factor I, CD5 and CD6 is known that have the scavenger receptor cysteine-rich (SRCR) domains observed in the macrophage scavenger receptor (Resnick, D., Pearson, A. and Krieger, M., Trends. Biochem. Sci., 19, 5-8, 1994).

In contrast to cyclophilin-C binding protein and complement factor I being secretory proteins, speract receptor, CD5 and CD6 are known to be membrane-bound proteins. Among these, a protein binding to membrane-bound protein CD6 was found to be the activated leukocyte adhesive molecule (ALCAM), and its binding site was localized to a SRCR domain structure of CD6 (Whitney, G.S., Starling, G.C., Bowen, M.A., Modrell, B., Siadak, A.W. and Aruffo, A.J., J. Biol. Chem., 270, 18187-18190, 1995).

Moreover, ALCAM, which is a ligand of CD6, is known to be expressed by activated lymphocytes and neurons, while CD6 is surmised to fulfill a certain regulatory function for maintaining homeostasis in the immune system and nervous system by means of the interaction with ALCAM.

In this manner, proteins composed of multi-domain structures not only have characteristic functions associated with each domain, but also are considered to function by having specific recognition functions interacting with each domain function.

### Disclosure of the Invention

In consideration of the present circumstances as described above, the object of the present invention is to provide a novel serine protease, and a novel serine protease DNA that codes for it. Moreover, another object of the present invention is to provide a process for producing a large amount of said protease using said DNA, and a process for screening physiologically active substances using said serine protease or DNA that codes for it.

As a result of repeated earnest research, the inventors of the present invention isolated cDNA that codes for a novel functional protein by screening cDNA having a characteristic 5' translation region using a region preserved well in cDNA for the probe that codes for serine protease occurring in the brain, thereby leading to completion of the present invention.

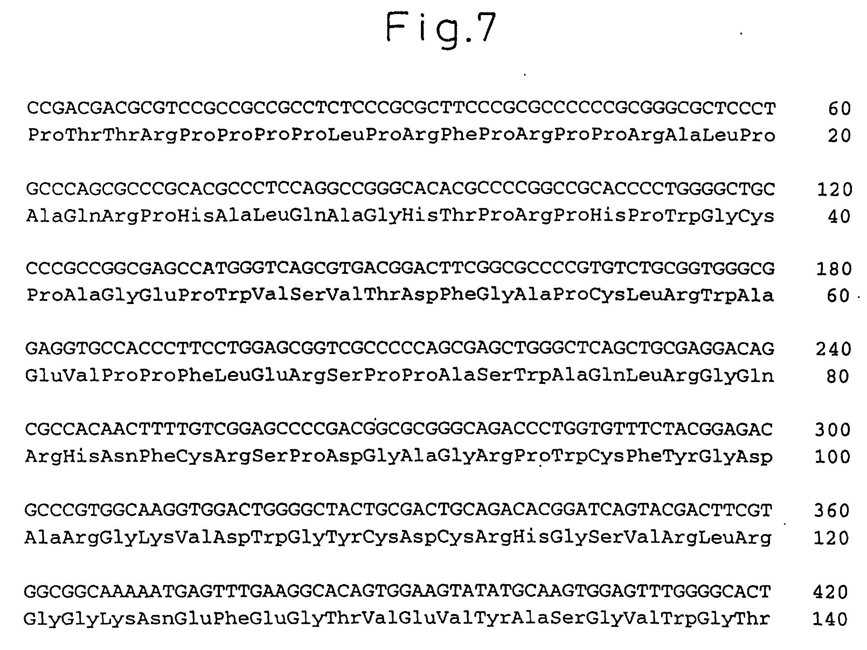

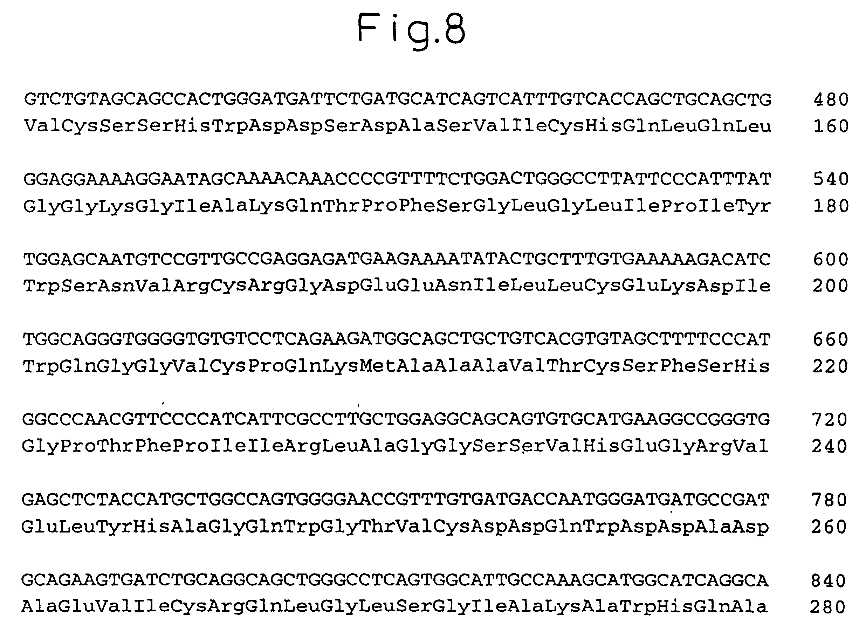

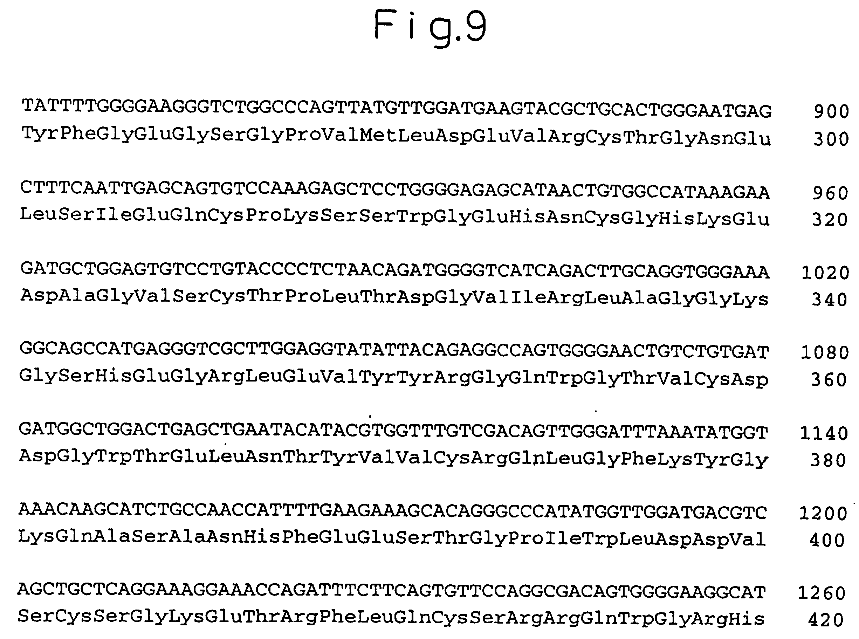

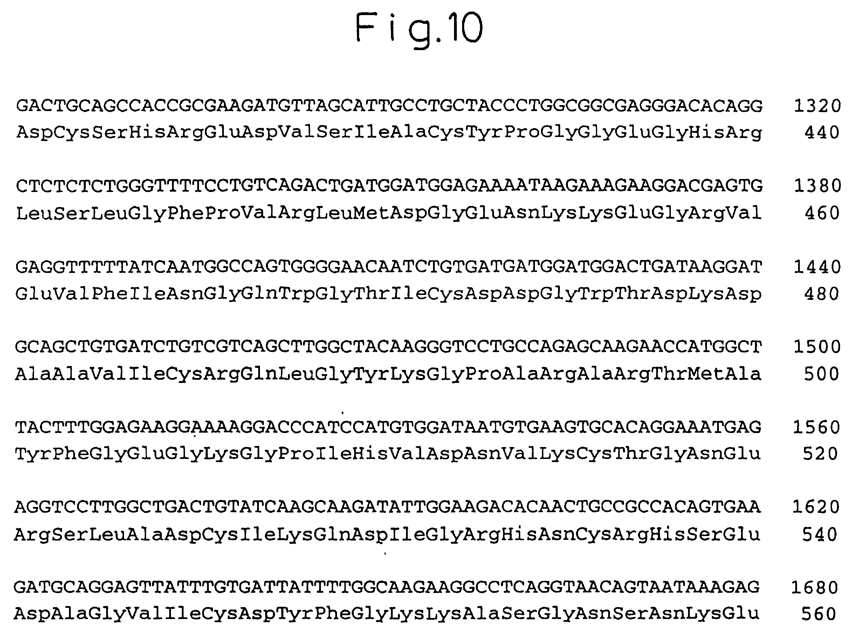

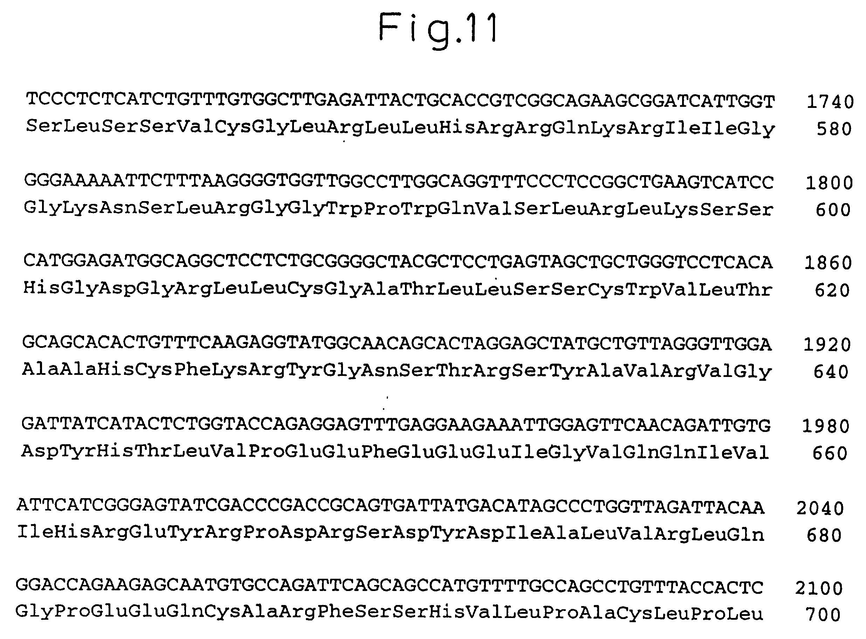

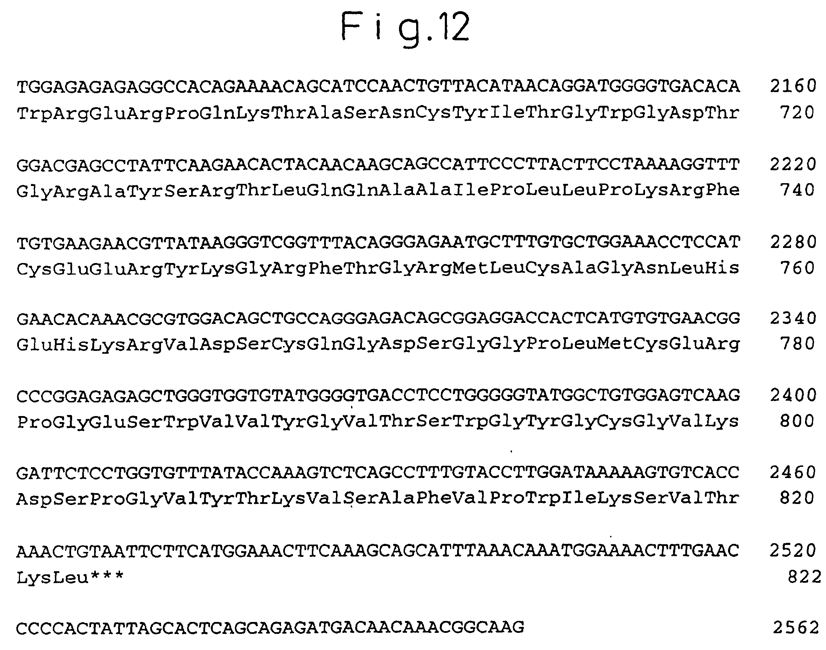

Thus, the present invention provides (1) a serine protease or its partial peptide containing an amino acid sequence identical to serine protease indicated in Figs. 7 to 12 (SEQ ID NO: 6), an amino acid sequence in which a portion of the identical amino acid sequence is deleted or substituted, or an amino acid sequence in which at least one amino acid is added to the identical amino acid sequence or an amino acid sequence in which a portion of the identical amino acid sequence is deleted or substituted.

Moreover, the present invention provides (2) a serine protease domain or its partial peptide containing an amino acid sequence identical to a serine protease domain comprising the amino acid sequence from amino acid no. 578 to 822 indicated in Figs. 7 to 12 (SEQ ID NO: 6), an amino acid sequence in which a portion of the identical sequence is deleted or substituted, or an amino acid sequence in which at least one amino acid is added to the identical amino acid sequence or an amino acid sequence in which a Portion of the identical amino acid sequence is deleted or substituted.

Moreover, the present invention provides (3) a kringle domain or its partial peptide containing an amino acid sequence identical to a kringle domain comprising the amino acid sequence from amino acid no. 40 to 112 indicated in Figs. 7 to 12 (SEQ ID NO: 6), an amino acid sequence in which a portion of the identical sequence is deleted or substituted, or an amino acid sequence in which at least one amino acid is added to the identical amino acid sequence or an amino acid sequence in which a portion of the identical amino acid sequence is deleted or substituted.

Moreover, the present invention provides (4) a scavenger receptor cysteine-rich (SRCR) domain or its partial peptide containing an amino acid sequence identical to an SRCR domain comprising the amino acid sequence from amino acid no. 117 to 217, from amino acid no. 227 to 327, from amino acid No. 334 to 433, or from amino acid No. 447 to 547 indicated in Figs. 7 to 12 (SEQ ID NO: 6), an amino acid sequence in which a portion of the identical sequence is deleted or substituted, or an amino acid sequence in which at least one amino acid is added to the identical amino acid sequence or an amino acid sequence in which a portion of the identical amino acid sequence is deleted or substituted.

Moreover, the present invention provides (5) DNA which codes for the serine protease, domain or their partial peptides as set forth in any one of the above-mentioned (1) to (4).

Moreover, the present invention provides (6) DNA which codes for a peptide having serine protease, domain or their partial peptide activity, and is hybridizable with DNA that codes for the serine protease, domain or their partial peptides as set forth in any one of the above-mentioned (1) to (4) under stringent conditions.

Moreover, the present invention provides (7) an expression vector containing the DNA as set forth in the above-mentioned (5) or (6).

Moreover, the present invention provides (8) a host transformed by the expression vector as set forth in the above-mentioned (7).

Moreover, the present invention provides (9) a process for preparing of serine protease, domain or their partial peptides comprising culturing or breeding the host as set forth in the above-mentioned (8), and harvesting serine protease, domain or their partial peptides.

Moreover, the present invention provides (10) an antibody whose antigen is the serine protease, domain or their partial peptides as set forth in any one of the above-mentioned (1) to (4).

Moreover, the present invention provides (11) a process for screening physiologically active substances that uses the serine protease, domain or their partial peptides as set forth in any one of the above-mentioned (1) to (4), or the DNA as set forth in the above-mentioned (5) or (6).

### Brief Description of the Drawings

Fig. 1 indicates a portion of the nucleotide sequence of cDNA that codes for mouse serine protease, and its corresponding amino acid sequence.
Fig. 2 indicates a portion of the nucleotide sequence of cDNA that codes for mouse serine protease, and its corresponding amino acid sequence.
Fig. 3 indicates a portion of the nucleotide sequence of cDNA that codes for mouse serine protease, and its corresponding amino acid sequence.
Fig. 4 indicates a portion of the nucleotide sequence of cDNA that codes for mouse serine protease, and its corresponding amino acid sequence.
Fig. 5 indicates a portion of the nucleotide sequence of cDNA that codes for mouse serine protease, and its corresponding amino acid sequence.
Fig. 6 indicates a portion of the nucleotide sequence of cDNA that codes for mouse serine protease, and its corresponding amino acid sequence.
Fig. 7 indicates a portion of the nucleotide sequence of cDNA that codes for human serine protease, and its corresponding amino acid sequence.
Fig. 8 indicates a portion of the nucleotide sequence of cDNA that codes for human serine protease, and its corresponding amino acid sequence.
Fig. 9 indicates a portion of the nucleotide sequence of cDNA that codes for human serine protease, and its corresponding amino acid sequence.
Fig. 10 indicates a portion of the nucleotide sequence of cDNA that codes for human serine protease, and its corresponding amino acid sequence.
Fig. 11 indicates a portion of the nucleotide sequence of cDNA that codes for human serine protease, and its corresponding amino acid sequence.
Fig. 12 indicates a portion of the nucleotide sequence of cDNA that codes for human serine protease, and its corresponding amino acid sequence.
Fig. 13 is an electrophoresis diagram indicating the results of Northern blotting that shows transcription of serine protease gene in various mouse organs.

### Mode for Carrying Out the Invention

Cloning of cDNA coding for mouse serine protease was performed by first preparing a cDNA library from mouse brain mRNA isolated and prepared in accordance with conventional methods, and then performing PCR using the cDNA library and PCR primers designed and prepared based on a serine protease motif. Using the resulting PCR product as a probe, clones were screened having a long 5' translation region and expected to code for a novel functional protein.

As a result, the inventors of the present invention succeeded in isolating a 2.7 kb cDNA named mouse BSSP-3. As a result of investigating the resulting cDNA sequence in accordance with conventional methods, mouse BSSP-3 cDNA was determined to code for a novel functional protein that contains not only a serine protease domain, but also a kringle domain and scavenger receptor cysteine-rich domains. The isolated mouse BSSP-3 cDNA coded for one Kringle domain, three scavenger receptor cysteine-rich domains, and one serine protease domain. A specific example is described in Example 1.

Next, when expression of mouse BSSP-3 mRNA was confirmed in various mouse organs and various sites of mouse brain using the entire length of the isolated mouse BSSP-3 cDNA as a probe, with respect to expression in various mouse organs, strong expression was observed particularly in the brain, while expression was also observed in the lung and kidney. In addition, with respect to various sites of mouse brain, strong expression was observed in the cerebrum and brain stem, and expression was also observed in the medulla oblongata. The size was only about 2.7 kb in all cases. Of the various sites in the brain that were examined, expression of mouse BSSP-3 mRNA was not observed in the cerebellum. A specific example is described in Example 2. Based on these findings, mouse BSSP-3 mRNA was confirmed to actually be expressed in mouse organs.

Moreover, as a result of screening the human brain cDNA library using mouse BSSP-3 cDNA as a probe, human BSSP-3 cDNA was able to be successfully isolated. As a result, the inventors of the present invention clearly showed that human BSSP-3 cDNA clearly differs from that which would be predicted from the primary structure of mouse BSSP-3 cDNA, and was determined to code for one kringle domain, four scavenger receptor cysteine-rich domains, and one serine protease domain. A specific example is described in Example 3. Moreover, when the inventors of the present invention expressed human BSSP-3 cDNA coding for serine protease mature protein in COS-1 cells, it was clearly determined to be a functional protein having enzyme activity. A specific example is described in Example 4.

Based on the above results, in terms of its primary structure, the mouse and human BSSP-3 cDNAs isolated here encode a novel functional protein that not only contain a novel serine protease domain, a novel kringle domain and novel scavenger receptor cysteine-rich domains, but also is functional proteins in which the serine protease domain has enzyme activity.

Not only is it clear that the novel functional protein in the present invention has complex functions due to its primary structure, but it also plays a certain role in the physiological function of the brain through the complex functions. Thus, the mouse BSSP-3 cDNA and novel functional protein encoded by the mouse BSSP-3 cDNA of the present invention provide useful means for pathological analysis of various types of mouse disease models. In addition, the human BSSP-3 cDNA and novel functional protein encoded by human BSSP-3 cDNA of the present invention also provide useful moans for screening therapeutic agents for various types of diseases based on useful information for disease treatment obtained through pathological analysis. Moreover, they can also be applied to the development of therapeutic drugs for actual human diseases.

Examples of these treatment methods include supplementary treatment by administration of the recombinant protein and the gene-expression promotion or inhibition therapy by the sense or antisense method. Moreover, each of the domain structures of the novel functional protein can also function independently. Thus, molecules that exhibit interaction with each domain structure can be identified after expressing each domain structure separately. In addition, by investigating the involvement in disease of the identified molecule group, supplementary treatment by administration of the recombinant protein and gene-expression promotion or inhibition therapy by the sense or antisense method can be applied.

The following provides an explanation of the present invention based on its examples.

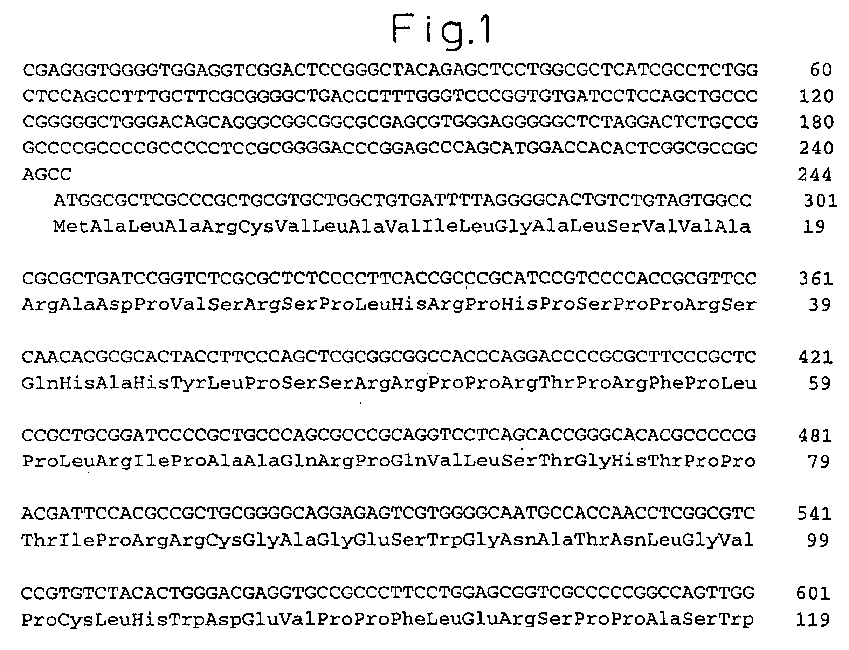

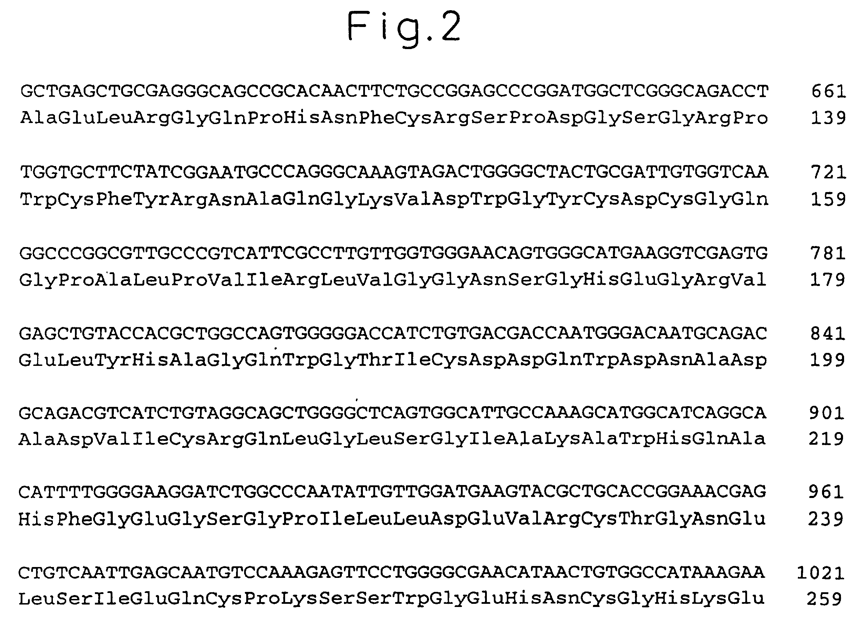

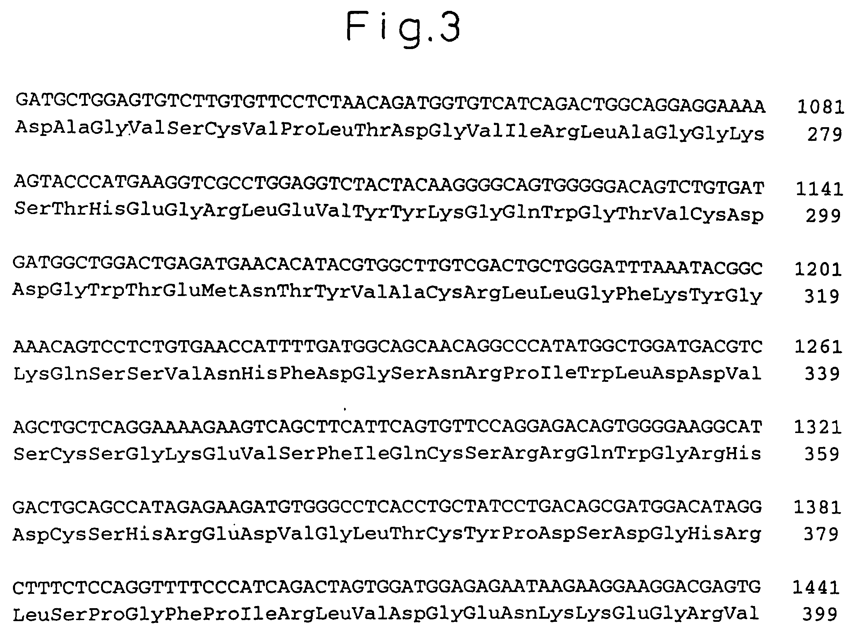

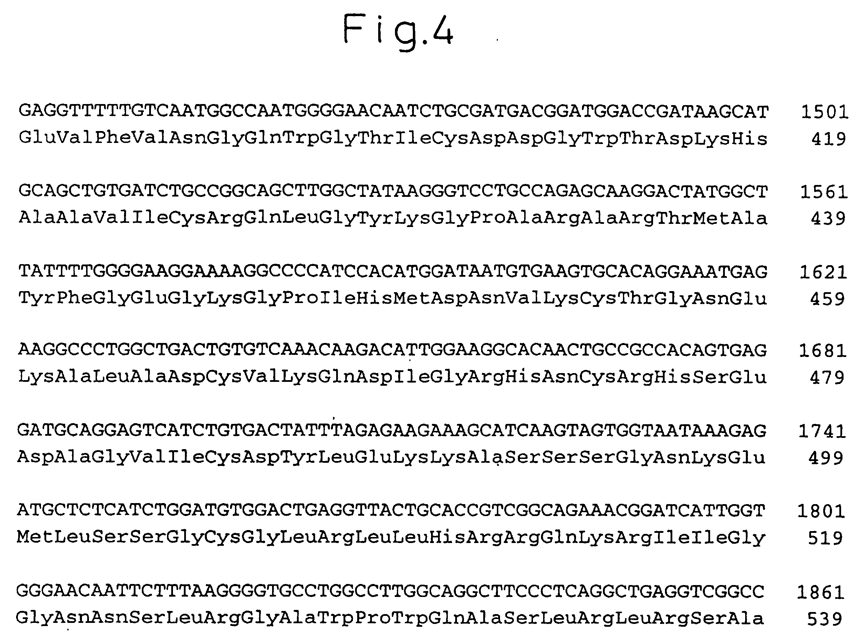

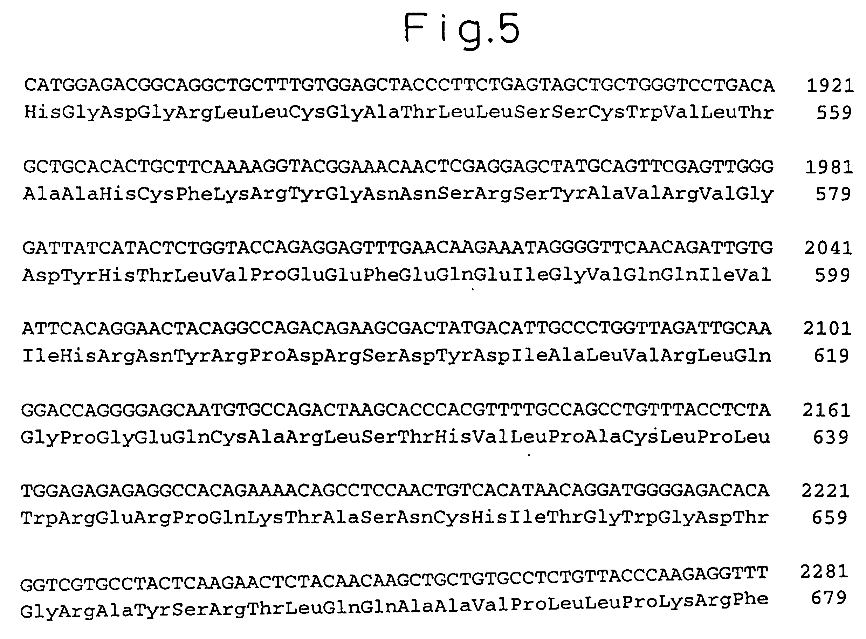

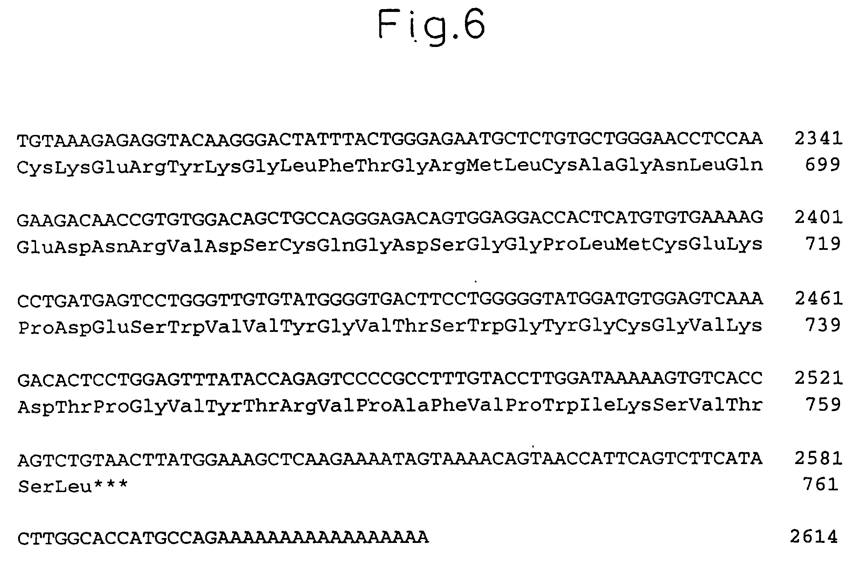

Although the present invention discloses the nucleotide sequence indicated in Figs. 1 to 6 (SEQ ID NO: 3) and Figs. 7 to 12 (SEQ ID NO: 5) as nucleotide sequences of DNA that code for novel serine proteases, the serine protease DNAs of the present invention are not limited to them. Once the amino acid sequence of naturally-occurring serine protease is determined, various nucleotide sequences that code for the same amino acid sequence can be designed based on codon degeneration and prepared. In this case, it is preferable to use codons that are used at high frequency in a host to be used.

In order to obtain DNA that codes for naturally-occurring serine protease of the present invention, although cDNA can be obtained in the manner described in the examples, it is not limited to this. Namely, once a single nucleotide sequence that codes for the amino acid sequence of naturally-occurring serine protease is determined, DNA coding for naturally-occurring serine protease can be cloned as cDNA by a strategy that differs from the strategy specifically disclosed in the present invention. Moreover, it can also be cloned from a genome of cells that produce it.

For example, the above-mentioned DNA can be cloned by the polymerase chain reaction (PCR) method using a DNA (nucleotide) primers as shown in Example 1.

The DNA of the present invention also codes for a protein or glycoprotein having serine protease activity, and includes DNA that hybridizes with the nucleotide sequence of Figs. 1 to 6 (SEQ ID NO: 3) or Figs. 7 to 12 (SEQ ID NO: 5). In addition, typical hybridization methods are well known among persons with ordinary skill in the art (examples of which include Experimental Medicine, special edition, Yodosha Publishing, "Biotechnology Experimental Method Series - Gene Engineering General Collection", Vol. 1.5, No. 11, pp. 24-60, 1987), and measurement of activity is also well known among persons with ordinary skill in the art.

In the case of cloning from a genome, the various primer nucleotides or probe nucleotides used in the examples can be used as probes for selecting genome DNA fragments. In addition, other probes can also be used that are designed based on the nucleotide sequence described in Figs. 1 to 6 (SEQ ID NO: 3) or Figs. 7 to 12 (SEQ ID NO: 5). Typical methods for cloning a target DNA from the genome are also well known among persons with ordinary skill in the art (Current Protocols in Molecular Biology, John Wiley & Sons, publisher, Chapters 5 and 6).

The DNA that codes for naturally-occurring serine protease of the present invention can also be prepared by chemical synthesis. DNA chemical synthesis can be easily performed by a person with ordinary skill in the art by using an automated DNA synthesizer such as the 396 DNA/RNA synthesizer of Applied Biosystems. Thus, a person with ordinary skill in the art can easily synthesize DNA of the nucleotide sequence indicated in Figs. 1 to 6 (SEQ ID NO: 3) or Figs. 7 to 12 (SEQ ID NO: 5).

A DNA that codes for naturally-occurring serine protease according to codons that differ from the native codons can also be prepared by chemical synthesis as mentioned above, and can also be obtained in accordance with conventional methods such as site-directed mutagenesis using a mutagenic primer with DNA or RNA having the nucleotide sequence indicated in Figs. 1 to 6 (SEQ ID NO: 3) or Figs. 7 to 12 (SEQ ID NO: 5) as a template (see, for example, Current Protocols in Molecular Biology, John Wiley & Sons, publisher, Chapter 8).

In this manner, once the amino acid sequence is determined, various variant forms of serine protease can be designed and produced, including polypeptides in which one or more amino acids are added to the naturally-occurring amino acid sequence while maintaining serine protease activity, polypeptides in which one or more amino acids are deleted from the above-mentioned naturally-occurring amino acid sequence while maintaining serine protease activity, polypeptides in which one or more amino acids in the above-mentioned naturally-occurring amino acid sequence are substituted with another amino acids while maintaining serine protease activity, and modified polypeptides in which the above-mentioned amino acid addition modification, amino acid deletion modification and amino acid substitution modification are combined, while maintaining serine protease activity.

Although there are no particular restrictions on the numbers of amino acids in the above-mentioned modification including amino acid addition, deletion or substitution modification, with respect to addition, the number of amino acids is dependent on the number of amino acids of known functional protein, e.g. maltose-binding protein, used to form a hybrid protein with the serine protease of the present invention for the purpose of extraction, purification or stabilization or on that of proteins having various physiological activities or the signal peptide added to the present serine protease. Namely, the number of amino acids to be modified is determined depending on the purpose of said modification, and for example, 1 to 50, and preferably 1 to 10, are added.

In addition, with respect to deletion, the number of amino acids that are deleted is designed and determined so as to maintain serine protease activity, and is, for example, 1 to 30, and preferably 1 to 20, or may be, for example, the number of amino acids in a region other than the active region of the present serine protease. Moreover, with respect to substitution, the number of amino acids that are substituted is designed and determined so as to maintain the serine protease activity, and is, for example, 1 to 10, and preferably 1 to 5.

In addition, the present invention provides a serine protease domain comprising the amino acid sequence from amino acid No. 517 to 761 or No. 578 to 822 indicated in Figs. 1 to 6 (SEQ ID NO: 4) or Figs. 7 to 12 (SEQ ID NO: 6), respectively, a kringle domain comprising the amino acid sequence from amino acid No. 85 to 157 or from No. 40 to 112 indicated in Figs. 1 to 6 (SEQ ID NO: 4) or Figs. 7 to 12 (SEQ ID NO: 6), respectively, or scavenger receptor cysteine-rich (SRCR) domains comprising the amino acid sequence from amino acid No. 166 to 266, from No. 273 to 372, from No. 386 to 486, from No. 117 to 217, from No. 227 to 327, from No. 334 to 433 or from No. 447 to 547 indicated in Figs. 1 to 6 (SEQ ID NO: 4) or Figs. 7 to 12 (SEQ ID NO: 6), respectively. Production of these domains can be performed by the method described later, a peptide synthesis method which itself is known, or by cleaving said serine protease by a suitable protease. In addition, modified domains that maintain the activity of the domains of the present invention or DNA that code for them can also be similarly produced.

When DNA of the serine protease or domain of the present invention is obtained in the manner described above, a recombinant serine protease or domain can be produced by ordinary gene recombination using the DNA for serine protease or domain. Namely, DNA coding for the serine protease or domain of the present invention is inserted into a suitable expression vector, said expression vector is introduced into suitable host cells, said host cells are cultured, and the target serine protease or domain is recovered from the resulting culture (cells or medium).

The serine protease or domain of the present invention may be obtained in a biochemically or chemically modified form, such as acylation of its N-terminal, including formylation, acetylation or other C₁₋₆ acylation or deletion. The secretion efficiency and expression level of the expression system may be improved by addition or modification of a signal sequence, or selection of host. Examples of addition and modification of signal sequence include a method in which a gene coding for a signal peptide of another structural peptide is ligated upstream of the 5'-end of the structural gene of the serine protease or domain of the present invention through a gene coding for a cleavable partial peptide. Specific examples of this include methods using the signal sequence of the trypsin gene and using a gene coding for an enterokinase recognition sequence, as described in Example 4.

Prokaryotic or eukaryotic organisms can be used for the host. Examples of prokaryotes that can be used include bacteria, and particularly Escherichia coli and the genus Bacillus such as Bacillus subtilis. Examples of eukaryotes that can be used include yeast such as the genus Saccharomyces such as Saccharomyces cerevisiae, and other eukaryotic microorganisms, insect cells such as armyworm cells (Snodoptera frugiperda), cabbage looper cells (Trichoplusia ni) and silkworm cells (Bombyx mori), and animal cells such as human cells, monkey cells and mice cells, specific examples of which include COS-1 cells, Vero cells, CHO cells, L cells, myeloma cells, C127 cells, BALB/c3T3 cells and Sp-2/O cells. Organisms themselves can also be used in the present invention, including insects such as cabbage looper and silkworm.

Examples of expression vectors that can be used include plasmids, phages, phagemids and viruses (Baculovirus (insects), Vaccinia virus (animal cells)) etc. A promoter in an expression vector is selected dependent on the host cells, and examples of bacterial promoters that are used include lac promoter and trp promoter, while examples of yeast promoters that are used include adhI promoter and pqk promoter.

In addition, examples of insect promoters include Baculovirus polyhedrin promoter, while examples of animal cell promoters include Simian Virus 40 early or late promoter, CMV promoter, HSV-TK promoter and SRα promoter. In addition, it is preferable to use an expression vector containing an enhancer, splicing signal, poly A addition signal, selective marker (such as dihydrofolate reductase gene (methotrexate-resistant) or neo gene (G418-resistant) in addition to those indicated above. Furthermore, in case of using an enhancer, SV40 enhancer, for example, is inserted upstream or downstream from the gene.

Transformation of host with an expression vector can be performed in accordance with conventional methods well known in the art, and these methods are described in, for example, Current Protocols in Molecular Biology, John Wiley & Sons, publisher. Culturing of the transformant can also be performed in accordance with conventional methods. Purification of serine protease or domain from the culture can be performed in accordance with conventional methods for isolation and purification of proteins, examples of which include ultrafiltration and various types of column chromatography such as chromatography using Sepharose.

Since the serine protease or domain of the present invention thus obtained is a functional protein, it provides a useful means for pathological analysis, allows screening of physiologically active substances using this protein, and is useful in research searching for therapeutic agents for various diseases. As a specific example of a screening method, screening for example of serine protease inhibitor, can be performed in the same manner as Example 4 by measuring a physiological activity of a tested sample, for example, a naturally-occurring component such as a peptide, protein, non-peptide compound, synthetic compound or fermentation product or compounds obtained from the culture supernatant of various cells, or artificial component an such as various types of synthetic compounds.

In addition, the above-mentioned measurement of physiological activity, measurement of binding affinity and so forth using the serine protease, domain or their partial peptides of the present invention or hosts transformed by DNA coding for the above-mentioned serine protease, domain or their partial peptides or its cell membrane fraction are also preferable embodiments of the screening method of the present invention.

In addition, DNA coding for the serine protease, domain or their partial peptides of the present invention is provided as a useful means of supplementary therapy by administration of the recombinant protein, the gene-expression promotion or inhibition therapy using the sense or antisense method, and elucidation of physiological functions within the body, and is also used for screening of new drugs based on the resulting information.

Moreover, the serine protease, domain or their partial peptides of the present invention, or DNA coding for them can be provided as a kit in a form that can be used when carrying out the above-mentioned screening methods.

Examples of partial peptides include peptide fragments comprising specific region of serin protase of the present invention such as peptide fragments present in the vicinity of a serine residue of an active site as well as peptide fragments that can be antibody recognition sites specific for the serine protease or domain of the present invention. Furthermore, production of said partial peptides can be performed by the methods previously described with respect to the serine protease or domain of the present invention, a peptide synthesis method which is itself known, or by cleaving said serine protease or domain with a suitable protease.

In addition, the above-mentioned cell membrane fraction refers to the fraction containing a large amount of cell membrane obtained after culturing host cells that allow expression of DNA coding for the serine protease, domain or its partial peptides of the present invention, under conditions that allow expression, and disrupting the resulting host cells containing serine protease, domain or its partial peptides by a method which is itself known.

A screening method for physiologically active substances using the serine protease, domain or its partial peptides of the present invention is performed by screening samples to be tested using the serine protease, domain or its partial peptides of the present invention, DNA coding for them, host cells containing said serine protease, domain or its partial peptides, or its cell membrane fraction. As a specific example of such method, screening is performed by measuring activity or measuring binding affinity using a substrate of the serine protease, domain or its partial peptides of the present invention, examples of which include a synthetic substrate such as a color-development substrate, or a substrate labeled with a radioisotope.

Furthermore, in the case of using host cells containing serine protease, domain or their partial peptides, the cells can be used after fixing with a known method (with glutaraldehyde, formaldehyde, etc.). In addition, in the case of using DNA coding for said serine protease, domain or their partial peptides, a technique for evaluating promotion or inhibition of gene expression can be performed using a reporter gene such as luciferase gene.

### Examples

### Example 1. Cloning of Novel Serine Protease Motif cDNA for Use as a Probe

### (1) PCR Using a Serine Protease Conservative Region

Preparation of mouse brain mRNA was performed using an RGT-T-primed first-strand kit (Pharmacia) in accordance with the attached instructions. 2 µl (1 µg) of oligo-dT primer was added to 5 µl (about 6 µg) of the resulting mRNA and heated for 10 minutes at 70°C followed by cooling rapidly on ice.

4 µl of 5x first strand buffer (250 mM Tris-HCl, pH 8.3, 375 mM KCl, 15 mM MgCl₂), 1 µl of 10 mM dNTP, 2 µl of 0.1 M DTT, diethylpyrocarbonate (DEPC)-treated distilled water and 5 µl (1000 U) of Super script IIRT were added to the denatured mRNA, and allowed to react for 1 hour at 37°C. PCR was then performed using the serine protease conservative region and the resulting first strand cDNA as the template.

Oligomer KY185 (5'-GTG CTC ACN GCN GCB CAY TG-3') shown in SEQ ID NO: 1 and synthesized based on the amino acid conservative region in the vicinity of an active residue (His) (N-Val-Leu-Thr-Ala-Ala-His-Cys), and oligomer KY189 (3'-CCV CTR AGD CCN CCN GGC GA-5') shown in SEQ ID NO: 2 and synthesized based on the amino acid preservation region in the vicinity of an active residue (Ser) (N-Gly-Asp-Ser-Gly-Gly-Pro-Leu), were used as primers. After performing PCR using Taq DNA polymerase (Amersham), the PCR reaction solution was subcloned to pCRII vector (Invitrogen).

### (2) Isolation and Purification of Mouse Brain mRNA for Screening

Preparation of mouse brain mRNA was performed using the Fast Track mRNA Isolation Kit (Invitrogen) in accordance with the attached instructions. Namely, 15 ml of lysis buffer was added to the entire extracted mouse brain and homogenized immediately with a teflonhomogenizer. After passing the homogenized tissue through a 21 gauge injection needle three times using a syringe, it was placed in a 50 ml centrifuge tube and incubated for 1 hour in a water bath at 45°C.

After incubation, the homogenized tissue was centrifuged for 5 minutes at 4000 x g, and the resulting supernatant was transferred to another 50 ml centrifuge tube. After adding 950 µl of 5 M NaCl solution, the solution was again passed through a 21 gauge injection needle three times using a syringe. Next, 1 tablet of oligo(dT) cellulose was added to the solution and after allowing to swell for 2 minutes, the solution was slowly rocked for 1 hour. One hour later, the solution was centrifuged for 5 minutes at 2,000 x g and after aspirating off the supernatant, the precipitate was suspended in 20 ml of binding buffer followed by washing the centrifuged residue in 10 ml of binding buffer.

Next, the precipitate was washed three times with 10 ml of low salt washing solution. After the final washing, the oligo(dT) cellulose was suspended in 800 µl of low salt washing solution, placed in a spin column, and centrifugal washing was repeated three times for 10 seconds at 5000 x g. After washing, 200 µl of elution buffer were added followed by repeating centrifugation for 10 seconds at 5000 x g twice to obtain 400 µl of mRNA solution. mRNA was recovered from the mRNA solution by ethanol precipitation in accordance with conventional methods, and dissolved in 20 µl of DEPC-treated distilled water.

### (3) Screening from a cDNA Library

### 〈Step 1〉 Synthesis of cDNA

2 µl (1 µg) of oligo dT NotI primer was added to 5 µl (about 6 µg) of the mRNA obtained in Example 1, part (2), and heated for 10 minutes at 70°C followed by cooling rapidly on ice. 4 µl of 5x first strand buffer (250 mM Tris-HCl pH 8.3, 375 mM KCl, 15 mM MgCl₂), 1 µl of 10 mM dNTP, 2 µl of 0.1 M DTT, DEPC-treated distilled water and 5 µl (1000 U) of Super Script IIRT were added to this denatured mRNA and allowed to react for 1 hour at 37°C.

Next, 91 µl of DEPC-treated distilled water, 30 µl of 5x second strand buffer (100 mM Tris-HCl pH 6.9, 450 mM KCl, 23 mM MgCl₂, 0.75 mM β-NAD+, 50 mM (NH₄)₂SO₄), 3 µl of 10 mM dNTP, 1 µl (10 U) of E. coli DNA ligase, 4 µl (40 U) of E. coli DNA polymerase and 1 µl (2 U) of E. coli RNAase H were added to this reaction solution, and after reacting for 2 hours at 16°C, 2 µl (10 U) of T4 DNA polymerase was added and allowed to react for 5 minutes at 16°C.

Moreover, 10 µl of 0.5 M EDTA was added to this solution and after mixing, 150 µl of phenol:chloroform:isoamyl alcohol (25:24:1) was added. After stirring, the solution was centrifuged for 5 minutes at 15,000 rpm and the supernatant was recovered. 10 µl of 5 M KOAc and 400 µl of ethanol were added to the resulting supernatant followed by stirring and centrifuging for 10 minutes at 15,000 rpm. The precipitate obtained by centrifugation was washed with 500 µl of 70% ethanol and after gently air drying, was dissolved in 25 µl of DEPC-treated distilled water.

### 〈step 2〉 Addition of EcoRI Adapter

10 µl of 5 x T4 DNA linking buffer (250 mM Tris-HCl pH 7.6, 50 mM MgCl₂, 5 mM ATP, 5 mM DTT, 25% (W/v) PEG 8000), 10 µl (10 µg) of EcoRI adapter solution and 5 µl (5 U) of T4 DNA ligase were added to 25 µl of the double strand cDNA obtained in the previous step. After reacting for 16 hours at 16°C, 50 µl of phenol:chloroform:isoamyl alcohol (25:24:1) was added, stirred and centrifuged for 5 minutes at 15,000 rpm followed by recovery of the supernatant. 5 µl of 5 M KOAc and 125 µl of ethanol were added to the recovered supernatant and stirred. After cooling for 20 minutes at -80°C, the supernatant was centrifuged for 10 minutes at 15,000 rpm. The precipitate resulting from centrifugation was washed with 200 µl of 70% ethanol and after gently air-drying, was dissolved in 40 µl of DEPC-treated distilled water.

### 〈Step 3〉 Ligation with λgt 10

1 µl (50 ng) of λgt 10 (EcoRI fragment) was added to 3 ml of size-fractionated cDNA solution followed by the addition of 11 µl of DEPC-treated distilled water, 4 µl of 5 x T4 DNA linking buffer and 1 µl of 5 x T4 DNA ligase and allowing to react for 3 hours at room temperature. After extracting with phenol:chloroform:isoamyl alcohol (25:24:1), adding 5 µl (5 µg) of yeast tRNA, 5 µl of 5 M KOAc and 125 µl of ethanol and stirring, the mixture was cooled for 20 minutes at -80°C and centrifuged for 10 minutes at 15,000 rpm. The precipitate resulting from centrifugation was washed with 200 µl of 70% ethanol and after gently air-drying, was dissolved in 5 µl of TE (10 mM Tris-HCl pH 8.0, 1 mM EDTA).

### 〈Step 4〉 Packaging

The ligated cDNA obtained in step 3 was packaged using Gigapack Packaging Extracts (Stratagene). Namely, after adding 10 µl of Freeze-thaw Extract contained in the kit to 1 µl of 0.1 µg/µl ligated cDNA, 15 µl of Sonic Extract contained in the kit was immediately added and mixed well. After allowing to stand for 2 hours at room temperature, 500 µl of phage dilution buffer (100 mM NaCl, 10 mM MgSO₄, 50 mM Tris-HCl pH 7.5, 0.01% gelatin) was added followed by addition of 20 µl of chloroform. After mixing well, the mixture was centrifuged for 5 minutes at the room temperature at 15,000 rpm and the supernatant was recovered to obtain a phage solution. According to conventional methods, it was used to infect the host E. coli after titrating the phase solution.

### 〈Step 5〉 Library Screening

The DNA fragment obtained in Example 1, part (1) was labeled with α-³²P dCTP using the BcaBest DNA labeling kit (Takara) to prepare a probe. A cDNA library comprising approximately 400,000 clones obtained in the previous step was screened using this probe. As a result, the longest clone of the inserted DNA fragment, pUC18/mBSSP-3/1-1 was obtained from the approximately 400,000 clones.

The total length of the pUC18/mBSSP-3/1-1 cDNA was 2,597 base pairs, and consisted of a 5' non-translation region of 244 base pairs, a translation region of 2283 base pairs, and a 3' non-translation region of 70 base pairs. The translation region was determined to code for a novel functional protein containing not only a serine protease domain (amino acid No. 517 to 761), but also a kringle domain (amino acid No. 85 to 157) and three scavenger receptor cysteine-rich domains (amino acid No. 166 to 266: domain 1, amino acid No. 273 to 372: domain 2, and amino acid No. 386 to 486: domain 3). The nucleotide sequence and corresponding amino acid sequence of pUC18/mBSSP-3/1-1 cDNA are shown in Figs. 1 to 6 (SEQ ID NO: 3).

### Example 2. Examination of Expression Site of mBSSP-3 by Northern Blotting

Mouse brain total RNA was prepared using Trizol reagent (Life Technology) in accordance with the attached instructions. Namely, after extracting mouse cerebrum, brain stem, cerebellum and medulla oblongata, the tissues were immediately homogenized with a Polytron (Kinematica), and the tissues were lysed by addition of 10 volumes (approx. 3 ml) of Trizol reagent relative to tissue volume. Moreover, 600 µl of chloroform was added, followed by mixing and centrifuging for 15 minutes at 4°C and 15,000 rpm. After centrifugation, the aqueous phase was recovered and 1500 µl of isopropanol was added to the recovered aqueous phase, followed by mixing and centrifuging for 30 minutes at 4°C and 15,000 rpm.

After dissolving the resulting total RNA precipitate of each site of mouse brain in 400 µl of DEPC-treated distilled water, it was blotted onto a membrane filter in accordance with conventional methods. Next, pUC18/mBSSP-3/1-1 was digested with restriction enzyme EcoRI, followed by isolation and purification of an approximately 2.7 kbp DNA fragment to prepare a probe by labeling with α-³²P dCTP using the above-mentioned method.

After hybridizing this probe overnight at 55°C with the membrane filters blotted with the total RNA prepared from each of the mouse brain sites described above, and with membrane filters blotted with commercially available mRNA prepared from various organs (Clontech), each of the membrane filters was washed for 20 minutes at room temperature with 2 x SSC containing 1% SDS (150 mM NaCl, 15 mM sodium citrate), and then washed twice for 30 minutes at 65°C after changing to 0.1 x SSC and 0.1% SDS. The membrane filters were then exposed for 30 minutes on a BAS2000 imaging plate (Fuji Photo Film).

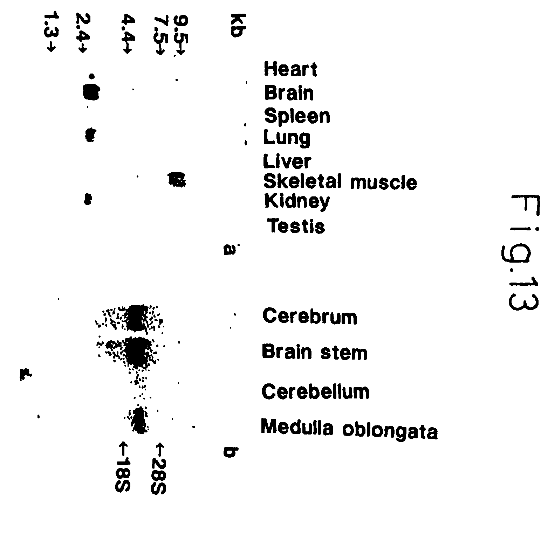

The results are shown in Fig. 13. With respect to expression in each organ, expression was confirmed in the brain, lung and kidney. With respect to each site of the brain, strong expression was observed in the cerebrum and brain stem. Although weak expression was also observed in the medulla oblongata, expression was not observed in the cerebellum. The expressed size was only about 2.7 kbp in all cases.

### Example 3 Cloning of Human BSSP-3 cDNA

Human brain cDNA library was purchased from Clontech. Mouse BSSP-3 cDNA fragment was fluorescent labeled using glutaraldehyde to prepare a probe. pUC18/hBSSP-3 was obtained as a result of screening the human brain cDNA library comprising approximately 400,000 clones using this probe.

The translation region of pUC18/hBSSP-3 cDNA was determined to code for a functional protein containing not only a serine protease domain (amino acid No. 578 to 822), but also a kringle domain (amino acid No. 40 to 112) and four scavenger receptor cysteine-rich domains (amino acid No. 117 to 217: domain 1, amino acid No. 227 to 327: domain 2, amino acid No. 334 to 433: domain 3, and amino acid No. 447 to 547: domain 4) in the same manner as mouse BSSP-3 cDNA.

However, it was clearly different from that predicted from the primary structure of mouse BSSP-3 cDNA. In contrast to mouse BSSP-3 having three scavenger receptor cysteine-rich domains, human BSSP-3 was determined to have four such domains. The nucleotide sequence and corresponding amino acid sequence of pUC18/hBSSP-3 are shown in Figs. 7 to 12 (SEQ ID NO: 5). pUC18/hBSSP-3 are shown in Figs. 7 to 12 (SEQ ID NO: 5).

### Example 4. Measurement of Enzyme Activity of Novel Serine Protease Mature Protein Coded by Human BSSP-3 cDNA

### (1) Construction of Expression Plasmid

pUC18/hBSSP-3 DNA fragment and pdKCR vector DNA fragment were ligated in accordance with conventional methods, E. coli JM109 was transformed, and the resulting colonies were analyzed by PCR to obtain the target serine protease hBSSP-3 expression plasmid pdKCR/hBSSP-3.

Next, primers were designed by amplifying genes coding for the signal sequence following the starting methionine of trypsin II and enterokinase recognition sequence so that EcoRI restriction enzyme recognition site was added upstream from the 5' side and BspMI restriction enzyme recognition site was added downstream from the 3' side. Using these primers, PCR was performed using pCR/Trypsin II plasmid for a template, and the product was digested with restriction enzymes (EcoRI and BspMI), followed by isolation and purification of an approximately 75 bp DNA fragment. Similarly, using a primer designed so that a BspMI restriction enzyme recognition site is added upstream from DNA coding for a mature protein of human BSSP-3, PCR was performed using pdKCR/hBSSP-3 for the template, followed by digestion of the product with restriction enzymes (BspMI and Bpu1102I) and isolation and purification of the DNA fragment.

Next, a resulting DNA fragment coding for trypsin II signal sequence and enterokinase recognition site, and a DNA fragment coding for human BSSP-3 mature protein were ligated into pdKCR/hBSSP-3 vector predigested with restriction enzymes (BspMI and Bpu1102I) in accordance with conventional methods, followed by transformation of E. coli JM109. Transformed colonies containing the target chimeric DNA were confirmed by PCR to obtain the expression plasmid (pdKCR/Trp-hBSSP-3).

### (2) Expression in COS-1 Cells

Chimeric gene DNA prepared in Example 4, part (1) was transfected into COS-1 cells using lipofectin (Life Technologies). Namely, 5 x 10⁵ COS-1 cells were grown in Dalvecco's minimum essential medium (DMEM, Nissui Pharmaceutical) containing 10% fetal bovine serum in 10 cm diameter culture dishes (Corning, 430167). On the following day, after rinsing the cells with 5 ml of Opti-MEM medium (Life Technologies), 5 ml of fresh Opti-MEM medium was added, followed by culturing for 2 hours at 37°C.

After culturing, a mixture of 1 µg of the above-mentioned plasmid and 5 µg of lipofectin was added to each dish, followed by culturing for 5 hours at 37°C. After culturing, 5 ml of Opti-MEM medium was added to make a total volume of 10 ml, followed by additional culturing for 72 hours at 37°C. After culturing, the culture supernatant was collected by centrifugation to prepare samples for measurement of enzyme activity. In addition, culture supernatant was prepared for use as a control by transfecting only expression plasmid pdKCR into COS-1 cells.

### (3) Measurement of Enzyme Activity

The enzyme activity in the culture supernatant obtained in Example 4, part (2) was measured. Namely, 5 µl of enterokinase (10 mg/ml, Biozyme Laboratories) was mixed with 45 µl of culture supernatant of COS-1 cells and allowed to react for 2 hours at 37°C. Next, 50 µl of 0.2 mM substrate solution prepared by dissolving synthetic substrate Boc-Phe-Ser-Arg-MCA (Peptide Research) in DMSO and diluted with 0.1 M Tris-HCl, pH 8.0 was added and allowed to react for 16 hours at 4°C. After reacting, fluorescence was measured at an excitation wavelength of 485 nm and fluorescent wavelength of 535 nm. As a result, enzyme activity was only observed when culture supernatant of COS-1 cells that expressed Trp-hBSSP-3 were digested with enterokinase.

Based on the above results, the serine protease domain of human BSSP-3 was determined to be a functional protein having enzyme activity.

### Effect of the Invention

The inventors of the present invention isolated mouse BSSP-3 cDNA from mouse brain cDNA library, that codes for a novel functional protein containing not only a novel serine protease domain, but also a novel kringle domain and novel scavenger receptor cysteine-rich domains. The isolated mouse BSSP-3 cDNA coded for 1 Kringle domain, 3 scavenger receptor cysteine-rich domains and 1 serine protease domain. In addition, as a result of examining the expression sites of the isolated mouse BSSP-3 mRNA, the inventors of the present invention determined that mouse BSSP-3 mRNA is strongly expressed in the brain, and particularly strongly in the cerebrum and brain stem.

Next, the inventors of the present invention succeeded at isolating human BSSP-3 cDNA from a human brain cDNA library using mouse BSSP-3 cDNA as a probe. As a result, the inventors of the present invention determined that human BSSP-3 cDNA is clearly different from that predicted from the primary structure of mouse BSSP-3 cDNA, in that it was determined to code for 1 kringle domain, 4 scavenger receptor cysteine-rich domains, and 1 serine protease domain.

Moreover, the inventors of the present invention determined that, when human BSSP-3 cDNA coding for serine protease mature protein was expressed in COS-1 cells, the expression product is a functional protein having enzyme activity. Not only was the novel functional protein in the present invention determined to have complex functions in terms of its primary structure, but that it plays a constant role in the physiological functions in the brain through the complex functions. Thus, the mouse BSSP-3 cDNA and novel functional protein encoded by the mouse BSSP-3 cDNA of the present invention provide useful means of pathological analysis of various types of mouse disease models.

In addition, the human BSSP-3 cDNA and novel functional protein encoded by the human BSSP-3 cDNA of the present invention provide means for screening therapeutic agents for various diseases based on the useful information for disease treatment obtained through the above pathological analysis. Moreover, they can also be applied to actual development of therapeutic drugs for human diseases. Examples of such treatment methods include supplementary therapy by administration of the recombinant protein and gene-expression promotion or inhibition therapy using the sense or antisense method.

Moreover, the structure of each domain of the novel functional proteins can also function independently. Thus, molecules that demonstrate interaction with each domain structure can be specified after separately expressing each domain structure. In addition, supplementary therapy by administration of the recombinant protein and the gene-expression promotion or inhibition therapy using the sense or antisense method can be performed by investigating the involvement of the specified molecular group in a disease.

## Claims

1. A serine protease or its partial peptide comprising an amino acid sequence identical to serine protease indicated in SEQ ID NO: 6, an amino acid sequence in which a portion of the identical amino acid sequence is deleted or substituted, or an amino acid sequence in which at least one amino acid is added to the identical amino acid sequence or an amino acid sequence in which a portion of the identical amino acid sequence is deleted or substituted.

2. A serine protease domain or its partial peptide comprising an amino acid sequence identical to a serine protease domain comprising the amino acid sequence from amino acid No. 578 to 822 indicated in SEQ ID NO: 6; an amino acid sequence in which a portion of the identical sequence is deleted or substituted, or an amino acid sequence in which at least one acid is added to the identical amino acid sequence or an amino acid sequence in which a portion of the identical amino acid sequence is deleted or substituted.

3. A kringle domain or its partial peptide comprising an amino acid sequence identical to a kringle domain comprising the amino acid sequence from amino acid No. 40 to 112 indicated in SEQ ID NO: 6, an amino acid sequence in which a portion of the identical sequence is deleted or substituted, or an amino acid sequence in which at least one amino acid is added to the identical amino acid sequence or an amino acid sequence in which a portion of the identical amino acid sequence is deleted or substituted.

4. A scavenger receptor cysteine-rich (SRCR) domain or its partial peptide comprising an amino acid sequence identical to an SRCR domain comprising the amino acid sequence from amino acid No. 117 to 217, from amino acid No. 227 to 327, from amino acid No. 334 to 433, or from amino acid No. 447 to 547 indicated in SEQ ID NO: 6, an amino acid sequence in which a portion of the identical sequence is deleted or substituted, or an amino acid sequence in which at least one amino acid is added to the identical amino acid sequence or an amino acid sequence in which a portion of the identical amino acid sequence is deleted or substituted.

5. DNA which codes for the serine protease, domain or their partial peptides as claimed in any one of the above-mentioned claims 1 to 4.

6. DNA which codes for a peptide having serine protease, domain or their partial peptide activity, and is hybridisable with DNA that codes for the serine protease, domain or their partial peptides as claimed in any one of the above-mentioned claims 1 to 4 under stringent conditions.

7. An expression vector containing the DNA as claimed in claims 5 or 6.

8. A host transformed by the expression vector as claimed in claim 7.

9. A process for preparing serine protease, domain or their partial peptides comprising culturing or breeding a host as claimed in claim 8, and recovering serine protease, domain or their partial peptides.

10. An antibody whose antigen is the serine protease, domain or their partial peptides as claimed in any one of claims 1 to 4.

11. A process for screening physiologically active substances that uses the serine protease, domain or their partial peptides as claimed in any one of claims 1 to 4, or the DNA as claimed in claims 5 or 6.
